Europäisches Patentamt

European Patent Office

Office européen des brevets

(19)

(11) **EP 1 141 230 B1**

(12) **EUROPÄISCHE PATENTSCHRIFT**

(45) Veröffentlichungstag und Bekanntmachung des Hinweises auf die Patenterteilung:
**29.01.2003 Patentblatt 2003/05**

(51) Int Cl.[7]: **C12M 3/00**, C12N 13/00, C12M 1/42

(21) Anmeldenummer: **99953355.7**

(22) Anmeldetag: **19.05.1999**

(86) Internationale Anmeldenummer:
**PCT/EP99/03442**

(87) Internationale Veröffentlichungsnummer:
**WO 99/061594 (02.12.1999 Gazette 1999/48)**

(54) **VERFAHREN UND VORRICHTUNG ZUR PERMEATION BIOLOGISCHER OBJEKTE**

METHOD AND DEVICE FOR PERMEATING BIOLOGICAL OBJECTS

PROCEDE ET DISPOSITIF POUR LA PERMEATION D'OBJETS BIOLOGIQUES

(84) Benannte Vertragsstaaten:
**AT BE CH CY DE DK ES FI FR GB GR IE IT LI LU MC NL PT SE**

(30) Priorität: **22.05.1998 DE 19823047**

(43) Veröffentlichungstag der Anmeldung:
**10.10.2001 Patentblatt 2001/41**

(73) Patentinhaber: **Evotec OAI AG
22525 Hamburg (DE)**

(72) Erfinder:
• **FUHR, Günter**
**D-13187 Berlin (DE)**
• **ZIMMERMANN, Ulrich**
**D-97295 Waldbrunn (DE)**
• **HAGEDORN, Rolf**
**D-13057 Berlin (DE)**

(74) Vertreter: **Hertz, Oliver, Dr. et al
v. Bezold & Sozien,
Akademiestrasse 7
80799 München (DE)**

(56) Entgegenhaltungen:
EP-A- 0 126 389          EP-A- 0 710 718
WO-A-98/56893          DE-A- 4 411 333

• **PATENT ABSTRACTS OF JAPAN vol. 012, no. 270 (C-515), 27. Juli 1988 (1988-07-27) & JP 63 049071 A (SHIMADZU CORP), 1. März 1988 (1988-03-01)**
• **PATENT ABSTRACTS OF JAPAN vol. 010, no. 296 (C-377), 8. Oktober 1986 (1986-10-08) & JP 61 111680 A (HITACHI LTD), 29. Mai 1986 (1986-05-29)**
• **PATENT ABSTRACTS OF JAPAN vol. 010, no. 128 (C-345), 13. Mai 1986 (1986-05-13) & JP 60 251877 A (HITACHI SEISAKUSHO KK), 12. Dezember 1985 (1985-12-12)**
• **PATENT ABSTRACTS OF JAPAN vol. 010, no. 128 (C-345), 13. Mai 1986 (1986-05-13) & JP 60 251874 A (HITACHI SEISAKUSHO KK), 12. Dezember 1985 (1985-12-12)**

Bemerkungen:
Derzeit sind die WIPO-Publikationsdaten A3 nicht verfügbar.

EP 1 141 230 B1

## Beschreibung

Die Erfindung betrifft ein Verfahren zur Manipulierung oder Bearbeitung biologischer Objekte mittels elektrischer Pulse, insbesondere zur Permeation und/oder Fusion von Zellen oder von synthetischen, membranumhüllten Gebilden wie Liposomen oder Vesikeln oder zur Permeation von Membran- oder Schichtmaterialien in Elektrodenstrukturen, und Vorrichtungen zur Durchführung des Verfahrens.

Bei vielen biotechnologischen, medizinischen oder genetischen Aufgaben ist die kurzzeitige und reversible Erhöhung der Durchlässigkeit der Umhüllung lebender, in einer Flüssigkeit suspendierter Zellen von Interesse (Übersicht in "Electromanipulation of Cells", U. Zimmermann, G.A. Neil, CRC, 1996). Neben chemischen und virusbasierten Methoden hat sich wegen der Einfachheit und Definiertheit der Applikation die Permeabilisierung mittels kurzer elektrischer Pulse durchgesetzt. Das Verfahren wird als Elektroporation, Elektropermeation oder auch Elektropermeabilisierung bezeichnet und mit kommerziellen Geräten von einer Vielzahl von Herstellern implementiert.

In herkömmlichen Elektroporationsgeräten befinden sich die zu manipulierenden Zellen in einer Lösung suspendiert in einer Küvette zwischen Elektroden zur Applikation der elektrischen Pulse. Allen Geräten gemeinsam ist der Einsatz eines oder mehrerer elektrischer Pulse einer Amplitude zwischen einigen 10 V bis zu einigen 1000 V. Die Pulsform ist geräteabhängig. Die Pulsdauer wird objektabhängig (vor allem größenabhängig) gewählt und liegt im Bereich von wenigen μs bis zu einigen 100 ms. Die Elektroden (zumeist zwei) werden jeweils paarig als Kathode bzw. Anode fest verschaltet.

Ein gravierendes, bisher ungelöstes Problem der Elektropermeabilisierung besteht darin, daß sich der pH-Wert in der Nähe der Elektroden durch zeitlich nicht stationäre Elektrolyseerscheinungen lokal ändert, so daß es zu nachhaltigen Störungen der Porationsbedingungen kommt. Je nach Lösungszusammensetzung führt die Beaufschlagung der Elektroden mit elektrischen Pulsen zur Erzeugung von $H^+$- (Protonen-) bzw. $OH^-$- (Hydroxylionen-) Anreicherungen ("Wolken") und somit zu einem lokal sauren oder basischen Bereich. Mit zunehmender Pulsdauer wandert von beiden Elektroden jeweils eine Front niedrigen bzw. hohen pH-Wertes aufeinander zu. Auch wenn die pH-Änderungen an beiden Elektrodenbereichen sich über die gesamte Flüssigkeitsfüliung der Küvette nahezu neutralisieren, sind die Zellen der jeweiligen räumlichen Bereiche einem pH-Gradienten bzw. -wechsel von bis zu 5 pH-Einheiten ausgesetzt, was zu einer erheblichen Beeinflussung der Lebensprozesse, bis zur Devitalisierung und Einleitung der Apoptose führen kann.

Außerdem werden durch die lokalen pH-Änderungen ggf. unerwünschte Elektrodenreaktionen ausgelöst. Wird beispielsweise Aluminium als Elektrodenmaterial verwendet, so kann beim Pulsen Aluminium gelöst werden und die Zellsuspension vergiften. Diesem Problem kann zwar durch den Einsatz edler Metalle als Elektrodenmaterialien begegnet werden, wodurch aber ein Elektroporationsgerät teuer wird.

Mit den bekannten Pulstechniken (Kondensatorentladung, Rechteckpuls, Dreieckpuls etc.) ist es nicht möglich, die pH-Belastung der Zellen zu verhindern, da es sich hierbei um grundlegende Erscheinungen an der Anode bzw. Kathode handelt. Es wird zwar bisher versucht, den Elektrolyseeffekt über die Applikation sehr kurzer Pulse zu minimieren. Dadurch wird jedoch die Einsetzbarkeit der Elektroporation eingeschränkt.

Gerade bei Objekten mit weiteren aufgelagerten Schichten, wie Bakterien, sind längere Pulszeiten jedoch wirksamer, so daß die pH-Änderungen besonders negativ zum Tragen kommen und ein Bedarf an alternativen Techniken ohne Verlust der Vorteile der elektrischen Feldapplikation besteht.

Aus EP 0 710 718 AI ist ein Verfahren zur Zellporation und -fusion unter Verwendung von elektrischen Hochfrequenzpulsen bekannt. Zur Puisbehandlung wird ein Elektrodenpaar mit Hochfrequenzspannungen beaufschlagt, wobei eine Elektrode des Elektrodenpaares auf Massepotential liegt, während an der anderen Elektrode ein Hochfrequenzwechselspannung angelegt wird.

Die Aufgabe der Erfindung ist es, ein gegenüber herkömmlichen Permeabilisierungssystemen verbessertes Verfahren auf der Basis der elektrischen Pulstechnik anzugeben, das sich insbesondere durch eine verminderte oder nahezu kompensierte pH-Änderungs-Belastung der Zellen und eine Unterdrückung von Elektrodenreaktionen auszeichnet, und eine Vorrichtung zur Durchführung des Verfahrens bereitzustellen, mit der die Einsatzmöglichkeiten der genannten Elektropermeationstechniken erweitert und die Effizienz der Permeation bzw. Zellfusion erhöht wird.

Diese Aufgabe wird durch ein Verfahren und Vorrichtungen mit den Merkmalen gemäß Patentanspruch 1, 5 bzw. 10 gelöst. Vorteilhafte Ausführungsformen der Erfindung ergeben sich aus den abhängigen Ansprüchen.

Der Erfindung liegt die Idee zugrunde, von der vorher festgelegten Beschaltung von Elektroden als Anode bzw. Kathode abzugehen und statt dessen die Polarität der Elektroden bzw. die Feldrichtung während der Pulszeit jeder Behandlung oder Poration mindestens einmal zu ändern. Dadurch werden an jeder Elektrode aufeinanderfolgend abwechselnd einander kompensierende elektrolytische Erhöhungen und Absenkungen des pH-Wertes erzielt. Beim Einsatz von z. B. zwei Elektroden wird jede Elektrode während der eingestellten Pulszeit (die in der Regel im μs- bis ms-Bereich liegt) mindestens einmal als Kathode und einmal als Anode betrieben. Um dies zu erreichen, wird der Puls in *sehr* rasch aufeinander folgende Einzel- oder Teilpulse entgegengesetzter Polarität zerlegt oder eine elektronische Torschaltung eingeführt, die es gestattet, ein elektrisches Wechselsignal als Pulspaket einzukoppeln oder die Verschaltung der Pulselektroden im µs-Bereich zu wechseln.

Der Begriff Kathode bzw. Anode bezeichnet hier jeweils die Elektrode mit dem positiveren bzw. negativeren Potential. Die Erfindung ist mit Potentialdifferenz-Pulsen unabhängig von deren absoluter Potentiallage realisierbar.

Die erfindungsgemäße Wechselpulstechnik basiert insbesondere auf den folgenden Überlegungen. Bei Applikation eines elektrischen Feld- oder Porationspulses einer vorbestimmten Pulszeit (z. B. 50 µs) für die Zellpermeation zwischen zwei Elektroden ist bei einer Zerlegung des Porationspulses in zwei Teilpulse unterschiedlicher Polarität (jeweils mit der halben Pulsdauer (z. B. 25 µs) oder mit einem anderen Teilerverhältnis), die im wesentlichen unmittelbar aufeinanderfolgen, jede der Elektroden einmal als Anode und Kathode wirksam. Dadurch werden Protonen- und Hydroxylionenwolken schneller aufeinanderfolgend erzeugt, als sie sich von den Elektroden entfernen können, so daß sie sich bereits innerhalb eines Bereiches der ersten Mikrometer um die Elektroden neutralisieren und dämpfen. Damit können die erzeugten pH-Änderungen für eine Zellbeeinflussung oder -schädigung nicht mehr oder nicht mehr in vollem Umfang wirksam werden.

Die Pulszerlegung (Teilerverhältnis, Amplitudenverhältnis, Formverhältnis) wird anwendungsabhängig unter Berücksichtigung der Diffusionszeiten und -längen der Protonen- und Hydroxylionen ausgewählt. Da die Diffusionszeiten und -längen in wäßrigen Lösungen abgeschätzt werden können, kann auch die Dauer der zerlegten Pulse über die bekannten Diffusionsgleichungen ermittelt werden. Hierfür ist der folgende Ansatz zu nutzen.

$$J_i = -D[dc_i/dx + ((z_iFc_i)/RT)^{\cdot} d\Psi/dx]$$

wobei $J_i$ der Flux des Ions i, D der Diffusionskoeffizient, c die Konzentration, x der Weg, z die Ladungszahl des Ions, $\Psi$ das Potential, T die Temperatur und F, R die Faraday- bzw. Gas-Konstanten sind. Für medizinisch und biologisch interessierende Flüssigkeiten ergeben sich bei Wegen kleiner 1 µm Zeiten im Bereich von einigen µs bis zu maximal 1 ms.

Die Dauer eines Teilpulses (Teilpulsdauer) wird so gewählt, daß sie kürzer oder vergleichbar mit der Diffusionszeit der beim jeweils vorhergehenden Teilpuls erzeugten $H^+$- oder $OH^-$-Ionenwolken ist. Damit wird eine räumlichen Trennung der Reaktionsprodukte ($H^+$- und $OH^-$-Ionenwolken) unterbunden. Da die Konzentration der $H^+$- oder $OH^-$-Ionenwolken lösungsabhängig unsymmetrisch sein kann, wird ggf. ein unsymmetrisches Teilerverhältnis der Teilpulsdauern realisiert. Die Schaltzeit zwischen den Teilpulsen ist vorzugsweise wesentlich kürzer als die Teilpulsdauern und beträgt rund 1 µs.

Eine Zerlegung der gewünschten Pulsdauer in mehr als zwei Einzel- oder Teilpulse ist insbesondere bei längeren oder mehreren Porationspulsen (ms-Bereich) sinnvoll und führt erfindungsgemäß zur Applikation einer Wechselspannung über eine elektronische Torschaltung. Entsprechend der oben erläuterten Diffusionslängen sind Frequenzen zwischen 1 kHz und 1 MHz, insbesondere 10 kHz bis 800 kHz, bevorzugt. Höhere Frequenzen erfordern eine deutliche Steigerung der Amplitude, um die Breakdownspannung der Zellmembranen zu übersteigen. Niedrigere Frequenzen sind in der Kürze der Pulsdauer nicht unterzubringen.

Die Erfindung bietet die folgenden Vorteile. Erfindungsgemäße Verfahren und Vorrichtungen können effektiv für die pHneutrale oder pH-kompensierte Permeabilisierung und Ein- bzw. Ausschleusung von Molekülen, Flüssigkeiten, Organellen und Mikroteilchen für biotechnologische, gentechnische und medizinische Anwendungen benutzt werden. Sie können gleichermaßen für Zellgruppen, die Zell-Zell-Fusion oder Aggregation von Zellen und Mikropartikeln oder Mikroorganismen verwendet werden. Das Verfahren macht es insbesondere möglich, Mikroelektroden und kleinste Räume (einige 10 bis 100 µm), wie sie in halbleitertechnologisch hergestellten Mikrosystemen erzeugt werden können, zu nutzen. Obwohl die zu behandelnden Objekte während jedes Pulses zeitlich wechselnden Feldrichtungen ausgesetzt werden, sind zuverlässige und reproduzierbare Ergebnisse wie bei der herkömmlichen Elektroporation erzielbar. Es werden Lösungsvergiftungen durch gelöstes Elektrodenmaterial vermieden. Damit lassen sich Porationsgefäße (insbesondere Küvetten) auch mit unedlen Elektrodenmetallen verwenden.

Bevorzugte Anwendungen der Erfindung sind die Bereitstellung pH-neutraler oder pH-kompensierter Elektroporationsgeräte und der Aufbau von Mikrosystemen für die Behandlung oder Manipulierung biologischer Zellen zwischen Mikroelektroden. Ein Mikrosystem hat charakteristische Dimensionen der Elektroden im Bereich von 100 µm oder geringer und charakteristische Dimensionen der Elektrodenabstände im Bereich von einigen (z. B. 2 bis 5) Zelldurchmessern.

Weitere Vorteile und Einzelheiten der Erfindung werden im folgenden unter Bezug auf die beigefügten Zeichnungen erläutert.
Es zeigen:

Fig. 1A,1B    eine Darstellung der erfindungsgemäßen Zerlegung eines Exponential-Porationspulses in Teilpulse,

Fig. 2A, 2B    eine Darstellung der erfindungsgemäßen Zerlegung eines Rechteck-Porationspulses in Teilpulse,

Fig. 3    eine Darstellung der erfindungsgemäßen Zerlegung eines Rechteck-Porationspulses in Teilpulse verschiedener Amplituden

Fig. 4      eine Darstellung der erfindungsgemäßen Zerlegung eines Rechteck-Porationspulses in eine Vielzahl von Teilpulsen,

Fig. 5      eine Darstellung der Applikation einer Wechselspannung für die Dauer eines Porationspulses,

Fig. 6      ein Blockschaltbild einer Steuerschaltung einer erfindungsgemäßen Porationsvorrichtung, und

Fig. 7      ein Blockschaltbild einer Steuerschaltung mit einer Torschaltung zur Applikation von Pulsen oder Wechselspannungen.

Die folgende Erläuterung bezieht sich auf die erfindungsgemäße Pulszerlegung, die bei Einführung entsprechender Maßnahmen bei herkömmlichen Elektroporationsgeräten implementiert werden kann. Auf bekannte Einzelheiten von Elektroporationsgeräten wie insbesondere die Eiektrodenanordnung, den Küvettenaufbau u. dgl. wird daher hier nicht gesondert eingegangen.

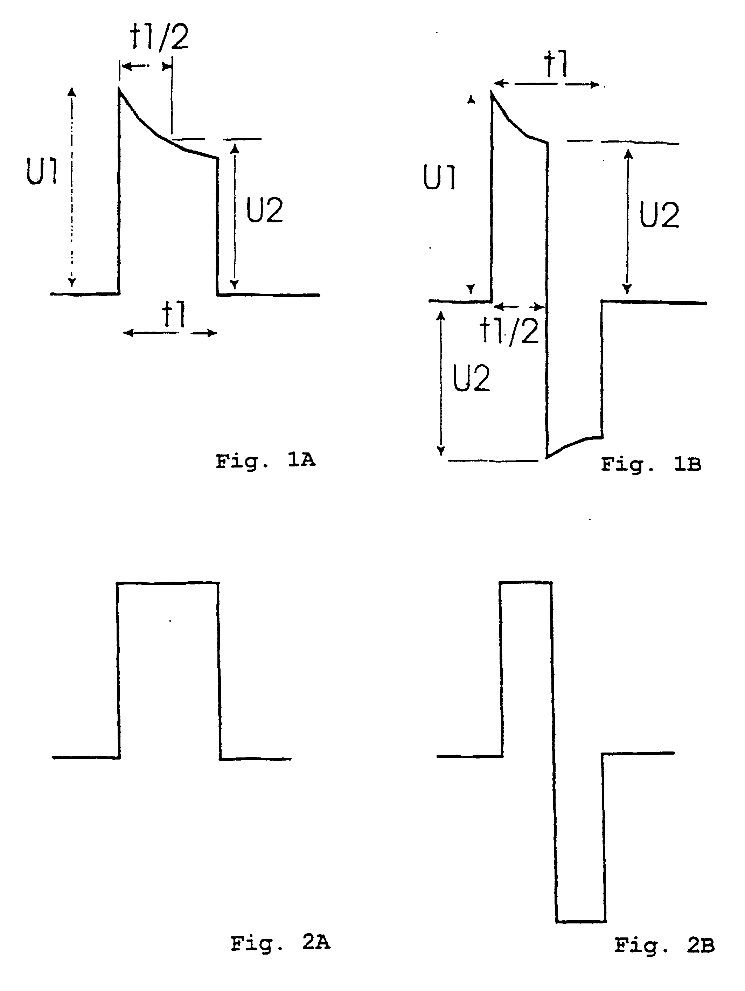

Fig. 1A zeigt den Spannungsverlauf einer zeitlich beschnittenen Kondensatorentladung, die bisher als Porationspuls an einem Elektrodenpaar appliziert wurde (Stand der Technik). Die Eingangsamplitude U1 fällt entsprechend einer Exponentialfunktion ab. Bei einer Pulszeit von t1, die in der Regel 10 μs bis zu einigen 100 ms beträgt, wird der Porationspuls abgeschaltet. Für die Dauer des Pulses erfolgen an der jeweiligen Anode und Kathode eine Ansäuerung bzw. Alkalisierung.

Fig. 1B zeigt den erfindungsgemäß zerlegten Puls als zeitlichen Spannungsverlauf zwischen zwei Elektroden beispielhaft mit zwei Teilpulsen unterschiedlicher Polarität. Die Kondensatorentladung fällt von der Eingangsamplitude U1 für die Teilpulsdauer t11 (hier t11 = 0.5 · t1) des ersten Teilpulses entsprechend einer Exponentialfunktion ab. Bei der Pulszeit t11, die in der Regel einige μs bis maximal 1 ms beträgt, wird der Teilpuls umgeschaltet. Für die restliche Zeit t12 (hier t11 = t12) wird die restliche Kondensatorentladung bis zur Endamplitude U2 mit umgekehrter Polarität an die Elektroden geschaltet. Abweichend vom hier angegebenen Teilerverhältnis t11 = t12 können andere Verhältnisse der Teilpulsdauern realisiert werden.

Es ist ersichtlich, daß bei den angegebenen Amplituden und Zeiten der Absolutwert der Flächen unter den beiden Pulsen der Fläche des Pulses in Fig. 1A vollständig entspricht. Durch die Verringerung der Amplitude bis zum Polarisationswechsel entsteht eine geringfügige Asymmetrie, die zu einer Rest-pH-Anderung führt. Dies kann durch eine Änderung des Teilerverhältnisses kompensiert oder, falls bei bestimmten Ionenzusammensetzungen der Medien auf der anodischen und kathodischen Seite nicht in gleicher Menge angesäuert bzw. alkalisiert wird, gezielt dazu genutzt werden, diese Differenzen auszugleichen.

Die genannte Asymmetrie bei der Verwendung von Entladevorgängen zur Pulserzeugung (asymmetrische Pulsformen) kann auch durch den Einsatz von Rechteckpulsen verhindert werden. Dies ist in den Fign. 2A und 2B illustriert. Fig. 2A zeigt den unzerlegten (herkömmlichen) Porationspuls. Erfindungsgemäß wird während des Pulsverlaufs die Polarität umgeschaltet (Fig. 2B) so daß sich zwei Teilpulse ergeben. Nunmehr ist die umgesetzte Leistung an jeder Elektrode exakt gleich. In entsprechender Weise sind auch anders realisierte Pulsformen zerlegbar.

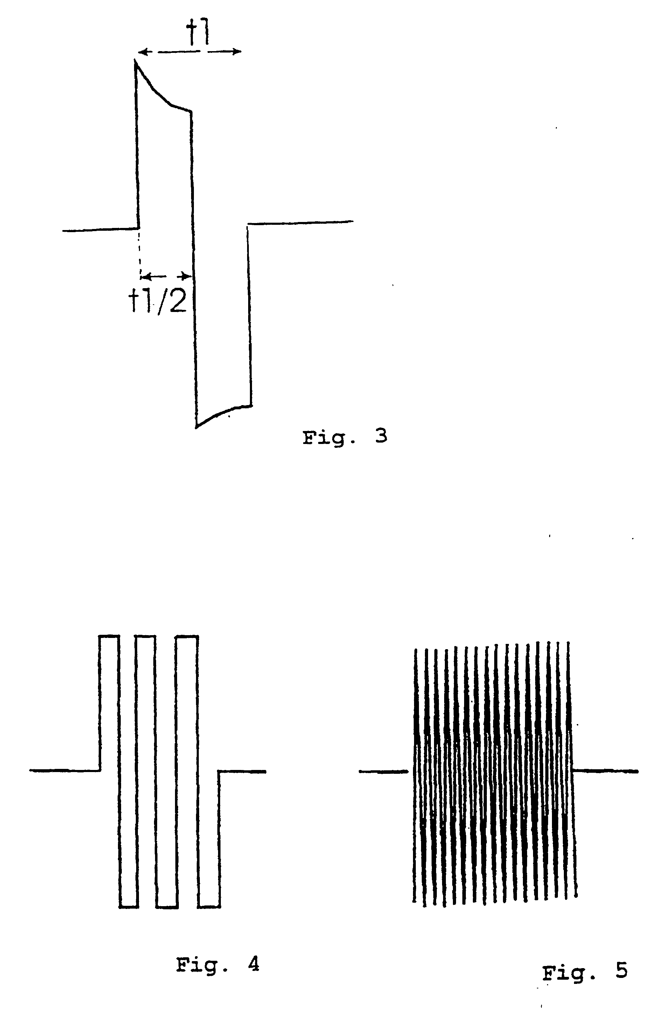

Die Zerlegung eines Porationspulses in Teilpulse unterschiedlicher Amplituden ist in Fig. 3 illustriert. Demnach ist z. B. die Amplitude des ersten Teilpulses geringer als die Amplitude des zweiten Teilpulses. Dies wird insbesondere bei an den Elektroden unsymmetrisch ablaufenden Elektrolyseprozessen bevorzugt, so beispielsweise in Chlorid-haltigen Suspensionslösungen, bei denen Chlor gasförmig lokal an einer Elektrode aus der Lösung entweichen kann. Die Amplitudenaufteilung erfolgt derart, daß die geringere $H^+$- bzw. $OH^-$-Bildung (pro Zeit) jeweils durch eine höhere Amplitude kompensiert und die Gesamtleistung des beabsichtigten Porationspulses beibehalten wird. Entsprechend kann zusätzlich die Teilpulsdauer variiert werden.

Fig. 4 zeigt die Zerlegung eines Porationspulses in mehr als zwei Teiipulse. Zur nahezu vollständigen Kompensation der pH-Effekte wird eine geradzahlige Zerlegung bevorzugt. Bei sehr vielen Zerlegungspulsen kann jedoch auch eine ungeradzahlige Aufteilung erfolgen, falls die Restdifferenz entsprechend klein bleibt bzw. die Medien eine Asymmetrie der Pulse erfordern.

Anstelle einer Pulsumschaltung an den Elektroden ist ersatzweise auch die Applikation einer Wechselspannung möglich. Fig. 5 zeigt die Applikation einer Vielzahl von Pulsen in Form einer Wechselspannung über ein für die gewünschte Dauer der Poration (entsprechend der Pulsdauer beim herkömmlichen Verfahren) freigegebenes elektronisches Tor.

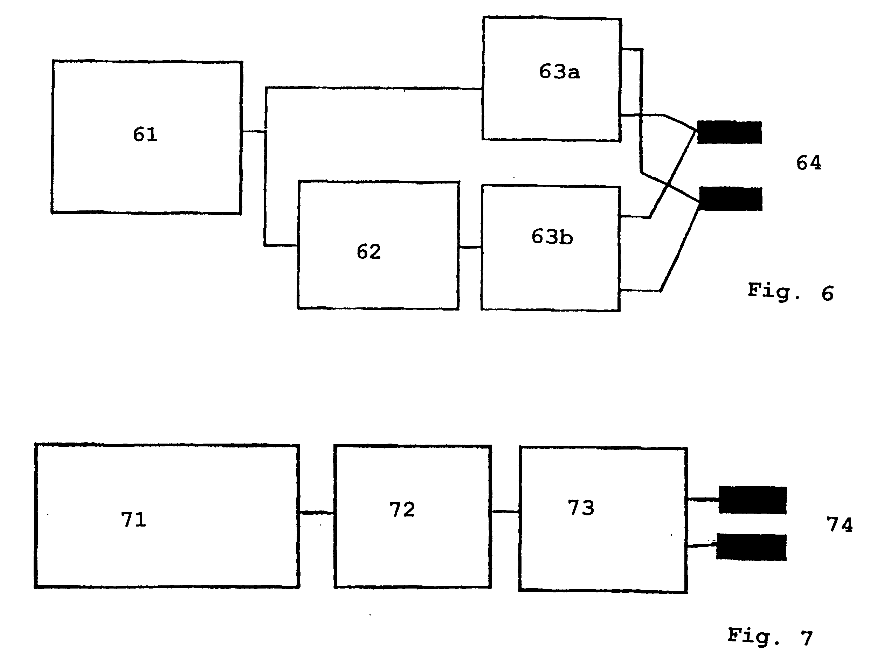

Ein Blockschaltbild einer elektronischen Schaltung, mit der die Pulszerlegung erfolgen kann, ist in Fig. 6 gezeigt. Das Bezugszeichen 61 steht für einen Pulsgenerator, der in herkömmlicher Weise die Einzelpulse erzeugt, hier jedoch jeweils mit der kürzeren Teilpulsdauer, die sich aus der Zerlegung der anwendungsabhängig gewünschten Pulszeit in 2, 4, 6 oder mehr Teilpulse ergibt. Der Pulsgenerator 61 ist einerseits über einen ersten Endverstärker 63a mit den Elektroden 64 und andererseits über ein Verzögerungsglied 62 und einen zweiten Endverstärker 63b mit den Elektroden 64 verbunden. Der zweite Endverstärker 63b ist ein Umkehrverstärker. Jeder Puls gelangt somit zuerst direkt an den Endverstärker 63a und an die Elektroden 64, und dann mit einer Zeitverzögerung über den Endverstärker 63a polaritätsumgekehrt an die Elektroden 64. Die Zeitverzögerung ist im wesentlichen gleich der Pulsbreite gewählt.

Die Endverstärker und das Verzögerungsglied erlauben eine Einstellung der jeweiligen Verstärkungsfaktoren (Amplitudenasymmetrie der Pulse) bzw. der Zeitverzögerung (Asymmetrie des Teilerverhältnisses der Pulse). Zusätzlich kann eine Pulsformschaltung vorgesehen sein. Die Teilpulse können insbesondere rechteckige, expotentielle, dreieckförmige, rampenförmige oder sinusförmige Pulsformen besitzen.

Soll eine Kondensatorentladung nachgeformt werden, ist der Pulsgenerator 61 entsprechend auszubilden. Eines von einer Vielzahl bekannter Verfahren ist die Digitalisierung des Pulses. Auf diese Weise kann eine Asymmetrie der Pulse zwischen dem Zweig 61 -> 63a und 61 -> 62 -> 63b erzeugt und damit jede beliebige Pulsform nachvollzogen werden.

Im Falle der Benutzung von sehr eng benachbarten Elektroden (z.B. in Mikrosystemen) sind Pulshöhen von maximal 100 V erforderlich, um eine Permeation von Zellen oder deren Fusion herbeizuführen. Dafür eignen sich eine Vielzahl von Schaltungsvarianten der Tri-State-Technik und Gegentaktstufen der Elektronik.

Fig. 7 zeigt ein Blockschaltbild zur Applikation der diskutierten Pulse oder Wechselspannungen über ein Tor. Das Bezugszeichen 71 bezeichnet einen Puls- oder Wechselspannungsgenerator, dessen Signal über eine Torschaltung 72 für die Zeit der gewünschten Applikation an den Endverstärker 73 gelangt und von dort an den Elektroden 74 zur Permeation oder Fusion der Zellen zur Verfügung steht.

Bei Anordnungen mit mehreren Elektrodenpaaren wird die erfindungsgemäße Pulszerlegung für jedes Elektrodenpaar durchgeführt. Die Schaltungen gemäß den Figuren 6 bzw. 7 werden entsprechend vervielfältigt bzw. angepaßt. Falls die Elektroporation mit mehreren Pulsen induziert wird, wird jeder der Pulse erfindungsgemäß zerlegt.

Die erfindungsgemäße Pulszerlegung ist bei der Behandlung biologischer beliebiger Objekte anwendbar, zu denen insbesondere biologische Objekte, wie beispielsweise biologische Zellen oder Zellgruppen, oder auch synthetische Objekte, wie beispielsweise Liposomen, Vesikel oder dgl., zählen.

**Patentansprüche**

1. Verfahren zur Behandlung biologischer oder synthetischer Objekte, die in einem Umgebungsmedium für eine vorbestimmte Pulszeit (t1) einem elektrischen Feld ausgesetzt werden, das durch mindestens zwei Elektroden gebildet wird, wobei während der Pulszeit (t1) jede der Elektroden mindestens einmal als Anode und mindestens einmal als Kathode angesteuert wird, so daß an jeder Elektrode ein Polaritätswechsel und dadurch abwechselnd elektrolytische Erhöhungen und Absenkungen des pH-Wertes erfolgen,

**dadurch gekennzeichnet, dass**

die Objekte während der Pulszeit (t1) einer Anzahl elektrischer Teilpulse, die jeweils bestimmte Teilpulsdauern besitzen, aufeinanderfolgend wechselnder Polarität oder Feldrichtung ausgesetzt sind, wobei die Teilpulse aufeinanderfolgend variierende Teilpulsdauern (t11, t12), Pulsformen und/oder Pulsamplituden besitzen und die Teilpulsdauern (t11, t12), Pulsfonnen und/oder Pulsamplituden jeweils so gewählt sind, dass durch die elektrolytischen Erhöhungen und Absenkungen des pH-Wertes im wesentlichen gleiche $H^+$- bzw. $OH^-$-Ionen-Konzentrationen erzeugt werden.

2. Verfahren gemäß Anspruch 1, bei dem die Teilpulsdauern so gewählt sind, daß die Erzeugung von $H^+$- oder $OH^-$-Ionenkonzentrationen an einer Elektrode während eines Teilpulses mindestens so schnell erfolgt wie die Diffusion der beim vorhergehenden Teilpuls erzeugten $OH^-$- oder $H^+$-Ionenkonzentrationen von der Elektrode in das Umgebungsmedium.

3. Verfahren gemäß einem der vorhergehenden Ansprüche, bei dem die Teilpulse rechteckige, exponentielle, dreieckförmige, rampenförmige oder sinusförmige Pulsformen besitzen.

4. Verfahren gemäß einem der vorhergehenden Ansprüche, bei dem eine Permeation und/oder Fusion von Zellen oder Zellgruppen oder von synthetischen, membranumhüllten Gebilden wie Liposomen oder Vesikeln oder eine Behandlung von membran- oder schichtförmigem Material erfolgt.

5. Vorrichtung zur Behandlung biologischer oder synthetischer Objekte in einem Umgebungsmedium zwischen mindestens zwei Elektroden, die mit einem Pulsgenerator verbunden sind

**dadurch gekennzeichnet, dass**

der Pulsgenerator mit den Elektroden über eine Steuerschaltung verbunden ist, die dazu eingerichtet ist, die Elektroden während einer vorbestimmten Pulszeit mit mindestens zwei Teilpulsen abwechselnd entgegengesetzter Polarität oder Feldrichtung zu beaufschlagen, wobei der Pulsgenerator dazu eingerichtet ist, die Teilpulse digitalisiert zur Erzeugung von Asymmetrien in der Pulshöhe, dem Pulsverlauf oder der Pulslänge bereitzustellen.

6. Vorrichtung gemäß Anspruch 5, bei der die Steuerschaltung einen ersten Endverstärker, über den der Pulsgenerator zur Erzeugung von Teilpulsen einer ersten Polarität mit den Elektroden verbunden ist, und einen zweiten Endverstärker mit einem Zeitverzögerungsglied umfaßt, über die der Pulsgenerator zur Erzeugung von Teilpulsen einer entgegengesetzten Polarität mit den Elektroden verbunden ist.

**7.** Vorrichtung gemäß Anspruch 5, bei der der Pulsgenerator mindestens einen Ladekondensator und die Steuerschaltung einen Wechselschalter umfaßt, mit dem während der Pulszeit die Kondensatorspannung abwechselnd auf eine der Elektroden geschaltet wird.

**8.** Vorrichtung gemäß Anspruch 5, bei der Pulsgenerator zur Erzeugung einer Wechsel- oder Tristate-Spannung eingerichtet ist und die Steuerschaltung eine Torschaltung umfaßt, die während der Pulszeit den Pulsgenerator mit den Elektroden verbindet.

**9.** Vorrichtung gemäß Anspruch 5, bei der der Pulsgenerator dazu eingerichtet ist, die Teilpulse digitalisiert in regelbarer Amplitude und/oder zur Nachbildung von beliebigen Signalformen bereitzustellen.

**10.** Elektroporationsgerät mit einer Elektropermeations- oder Fusionskammer, das eine Vorrichtung gemäß einem der Ansprüche 5 bis 9 enthält.

**11.** Elektroporationsgerät gemäß Anspruch 10, das als Mikrosystem mit einer Multielektrodenanordnung gestaltet ist.

**12.** Elektroporationsgerät gemäß Anspruch 11, bei dem das Mikrosystem charakteristische Dimensionen der Elektroden von 100 um oder geringer und charakteristische Dimensionen der Elektrodenabstände von einigen Zelldurchmessern besitzt.

**Claims**

**1.** Method for treating biological or synthetic objects which are subjected to an electrical field in a surrounding medium for a predetermined pulse time (t1) which field is formed by at least two electrodes, each of the electrodes being actuated during the pulse time (t1) at least once as anode and at least once as cathode, so that a polarity change is effected at each electrode and because of this electrolytic increases and decreases in the pH value are effected alternately,
**characterised in that,**
during the pulse time (t1), the objects are subjected to a number of electrical partial pulses which have specific partial pulse durations respectively and are of successively alternating polarity or field direction, the partial pulses having successively varying partial pulse durations (t11, t12), pulse forms and/or pulse amplitudes and the partial pulse durations (t11, t12), pulse forms and/or pulse amplitudes respectively being chosen such that, by means of the electrolytic increases and decreases in the pH value, essentially the same $H^+$ or $OH^-$ ion concentrations are produced.

**2.** Method according to claim 1, in which the partial pulse durations are chosen such that the production of $H^+$ or $OH^-$ ion concentrations at an electrode during a partial pulse is effected at least as rapidly as the diffusion of the $OH^-$ or $H^+$ ion concentrations, which were produced during the preceding partial pulse, by the electrode in the surrounding medium.

**3.** Method according to one of the preceding claims, in which the partial pulses have rectangular, exponential, triangular-shaped, ramp-shaped or sinusoidal pulse forms.

**4.** Method according to one of the preceding claims, in which a permeation and/or fusion of cells or cell groups or of synthetic, membrane-covered structures such as liposomes or vesicles or treatment of membrane- or layer-shaped material is effected.

**5.** Device for treating biological or synthetic objects in a surrounding medium between at least two electrodes which are connected to a pulse generator, **characterised in that**
the pulse generator is connected to the electrodes via a control circuit, which is equipped to apply to the electrodes at least two partial pulses alternately of an opposite polarity or field direction during a predetermined pulse time, the pulse generator being equipped to make the partial pulses available in a digitalised form in order to produce asymmetries in the pulse height, the pulse course or the pulse length.

**6.** Device according to claim 5, in which the control circuit comprises a first output amplifier, via which the pulse generator is connected to the electrodes in order to produce partial pulses of a first polarity, and a second output amplifier with a time delay member, via which the pulse generator is connected to the electrodes in order to produce partial pulses of an opposite polarity.

**7.** Device according to claim 5, in which the pulse generator comprises at least one charging capacitor and the control circuit comprises a change-over switch with which the capacitor voltage is alternately connected to one of the electrodes during the pulse time.

**8.** Device according to claim 5, in which the pulse generator is equipped to produce an alternating- or tristate voltage and the control circuit comprises a gate circuit, which connects the pulse generator to the electrodes during the pulse time.

9. Device according to claim 5, in which the pulse generator is equipped to make the partial pulses available in a digitalised form in adjustable amplitude and/or in order to reproduce any desired signal forms.

10. Electroporation apparatus with an electropermeation- or fusion chamber, which apparatus contains a device according to one of the claims 5 to 9.

11. Electroporation apparatus according to claim 10, which is configured as a microsystem with a multielectrode arrangement.

12. Electroporation apparatus according to claim 11, in which the microsystem has characteristic dimensions of the electrodes of 100 mm or less and characteristic dimensions of the electrode spacings of a few cell diameters.


**Revendications**

1. Procédé de traitement d'objets biologiques ou synthétiques qui sont soumis à un champ électrique dans un milieu pendant un temps d'impulsion prédéfini (t1), qui est créé par au moins deux électrodes, dans lequel pendant le temps d'impulsion (t1) chacune des électrodes est employée au moins une fois comme anode et au moins une fois comme cathode, de sorte que sur chaque électrode il se produise un changement de polarité et par là, alternativement, des augmentations et des baisses électrolytiques du pH, **caractérisé en ce que** les objets, pendant le temps d'impulsion (t1) sont soumis à un certain nombre d'impulsions électriques partielles qui possèdent chacune des durées d'impulsion partielle définie, et sont par la suite soumis à un changement de polarité ou à une orientation du champ, dans lequel les impulsions partielles possèdent des durées d'impulsion partielle (t11, t12), des formes d'impulsion et/ou des amplitudes d'impulsion consécutivement (t11, t12) variables, et les durées d'impulsion partielle les formes d'impulsion et/ou les amplitudes d'impulsion sont respectivement sélectionnées de sorte que les augmentations et les baisses électrolytiques du pH permettent de produire des concentrations d'ions $H^+$ ou $OH^-$ identiques pour l'essentiel.

2. Procédé selon la revendication 1, pour lequel les durées d'impulsion partielle sont sélectionnées de sorte que la production de concentrations d'ions $H^-$ ou $OH^-$ sur une électrode pendant une impulsion partielle se produise au moins aussi vite que la diffusion des concentrations d'ions $OH^-$ ou $H^+$ de l'électrode produites lors de l'impulsion partielle précitée créée dans le milieu.

3. Procédé selon l'une quelconque des revendications précédentes, dans lequel les impulsions partielles possèdent des formes d'impulsion rectangulaires, exponentielles, triangulaires, en forme de rampe ou de sinus.

4. Procédé selon l'une quelconque des revendications précédentes, dans lequel se produit une perméation et/ou une fusion de cellules ou de groupes de cellules ou de structures synthétiques ou membraneuses comme des liposomes ou des vésicules ou un traitement d'un matériau membraneux ou stratifié.

5. Dispositif de traitement d'objets biologiques ou synthétiques dans un milieu, entre au moins deux électrodes qui sont reliées par un générateur d'impulsions, **caractérisé en ce que** le générateur d'impulsions est relié aux électrodes par un circuit qui est agencé de sorte que les électrodes produisent pendant une durée d'impulsion prédéfinie au moins deux impulsions partielles de polarité ou d'orientation de champ alternativement opposée, dans lequel le générateur d'impulsions est agencé afin de produire les impulsions partielles de façon digitalisée pour créer des asymétries dans la grandeur, l'amplitude ou la longueur de l'impulsion.

6. Dispositif selon la revendication 5, dans lequel le circuit comprend un premier amplificateur final, par lequel le générateur d'impulsions est relié aux électrodes pour créer des impulsions partielles d'une première polarité, et un second amplificateur final doté d'un élément de décélération, par lequel le générateur d'impulsion est relié aux électrodes afin de créer des impulsions partielles de polarité opposée.

7. Dispositif selon la revendication 5, dans lequel le générateur d'impulsions comprend au moins un condensateur de charge et le circuit comprend un commutateur-inverseur, qui permet pendant la durée de l'impulsion de coupler la tension du condensateur alternativement à l'une ou l'autre des électrodes.

8. Dispositif selon la revendication 5, dans lequel le générateur d'impulsions est agencé afin de créer une tension alternée ou à trois états, et le circuit comprend une porte qui relie pendant le temps d'impulsion le générateur d'impulsions aux électrodes.

9. Dispositif selon la revendication 5, dans lequel le générateur d'impulsions est agencé afin de produire les impulsions partielles de façon numérisée dans une amplitude réglable et/ou afin de reproduire n'importe quelle forme de signal.

**10.** Appareil d'électroporation doté d'une chambre d'électroperméation ou d'une chambre de fusion, qui comprend un dispositif selon l'une quelconque des revendications 5 à 9.

**11.** Appareil d'électroporation selon la revendication 10, ayant l'aspect d'un micro-système avec un agencement multi-électrodes.

**12.** Appareil d'électroporation selon la revendication 11, dans lequel le micro-système possède des dimensions caractéristiques des électrodes de 100 μm ou moins, et des dimensions caractéristiques d'écartement des électrodes de quelques diamètres cellulaires.

Fig. 1A

Fig. 1B

Fig. 2A

Fig. 2B

Fig. 3

Fig. 4

Fig. 5

Fig. 6

Fig. 7